# EUROPEAN PATENT APPLICATION

(11) **EP 4 230 212 A1**
(43) Date of publication of application: **23.08.2023**
(21) Application number: 22157021.1
(22) Date of filing: 16.02.2022
(51) Int. Cl.: A61K 31/728, A61K 33/08, A61P 1/02

(54) **COMPOSITION AND KITS FOR THE PREVENTION OR TREATMENT OF GUM DISEASES**

(71) Applicant: UNIVERSITÄTSmedizin der Johannes Gutenberg- Universität Mainz, 55131 Mainz (DE)
(72) Inventor: MÜLLER-HEUPT, Lena Katharina, 64572 Büttelborn (DE); ECKELT, John, 55130 Mainz (DE)
(74) Representative: Patentanwälte Dr. Keller, Schwertfeger Partnerschaft mbB

(57) **Abstract**

The present invention relates to compositions comprising hyaluronic acid or a pharmaceutically acceptable salt thereof, and calcium peroxide (CaO₂) for use in the prevention or treatment of a gum disease. Furthermore, the invention relates to kits for delivering a composition into a gingival pocket, a periodontal pocket, or a pocket resulting from peri-implantitis, or for applying said composition on or into the oral mucosa.

## Description

### Field of the Invention

The invention relates to compositions comprising hyaluronic acid or a pharmaceutically acceptable salt thereof, and calcium peroxide (CaO₂) for use in the prevention or treatment of a gum disease. Furthermore, the invention relates to kits for delivering a composition into a gingival pocket, a periodontal pocket, or a pocket resulting from peri-implantitis, or for applying said composition on or into the oral mucosa.

### Background of the Invention

Gingivitis, periodontitis, and peri-implantitis are the most common biofilm-associated, inflammatory gum diseases in humans or mammals. Without treatment, these gum diseases result in tooth or implant loss. Periodontitis and peri-implantitis develop as a result of dysbiosis of the bacterial population in susceptible individuals, associated with a dysregulation of the immune-inflammatory response. Dysbiosis is thus responsible for the development of microbe-related pathologies and a host-mediated breakdown of connective tissue and alveolar bone (Darveau, R.P. Periodontitis: A polymicrobial disruption of host homeostasis. Nat. Rev. Microbiol. 2010, 8,481-490.). Periodontitis and peri-implantitis also involve the destruction of the connective tissue including collagens, proteoglycans, and other components of the extracellular matrix. Gingivitis is an inflammatory response of the gingival tissues to the metabolic products and pathogenic toxins of bacteria found in oral plaque.

The transition from periodontal health to disease is associated with a dramatic shift from a symbiotic microbial community, which is composed mostly of facultative bacterial genera such as *Actinomyces* and *Streptococci,* to a dysbiotic microbial structure composed mainly of anaerobic genera from the phyla *Firmicutes, Proteobacteria, Spirochaetes, Bacteroidetes* and *Synergistetes* (Hajishengallis G. Periodontitis: from microbial immune subversion to systemic inflammation. Nat Rev Immunol. 2015;15(1):30-44. doi:10.1038/nri3785.).

A gram-negative anaerobic bacterial flora induces a cascade of immune responses and thereby promotes tissue breakdown. Soft tissue and alveolar bone are consequently degraded, and the gram-negative anaerobes can grow under optimal conditions in the periodontal pockets. However, although the etiology of periodontal and peri-implant diseases is based on a dysbiotic microbial structure, and although some well-characterized pathogens display destructive virulence factors, the pathogenesis of periodontitis and peri-implantitis is essentially mediated by the host response (Van Dyke, T.E.; Serhan, C.N. Resolution of inflammation: A new paradigm for the pathogenesis of periodontal diseases. J. Dent. Res. 2003, 82, 82-90.0). There are different approaches for the treatment of gingivitis, periodontitis or peri-implantitis, most of them involve the application of anti-bacterial compositions (Lasserre, J.F.; Toma, S.; Bourgeois, T.; El Khatmaoui, H.; Marichal, E.; Brecx, M.C. Influence of low direct electric currents and chlorhexidine upon human dental biofilms. Clin. Exp. Dent. Res. 2016, 2, 146-154.).

In one common approach, a biodegradable chlorhexidine (CHX) chip is used as an adjunct to scaling and root planing (SRP) in the treatment of moderate to severe periodontitis patients (Medaiah S, Srinivas M, Melath A, Girish S, Polepalle T, Dasari AB. Chlorhexidine chip in the treatment of chronic periodontitis - a clinical study. J Clin Diagn Res. 2014;8(6):ZC22-ZC25. doi:10.7860/JCDR/2014/8808.4477). However, studies have shown that the use of CHX as an adjuvant after SRP has no significant positive effect on the microbiome and tissue attachment (Grisi, D.C., et al.,2002; Carvalho, J., et al., 2007), possibly because CHX is toxic to fibroblasts and osteoblasts, and thus inhibits tissue regeneration.

Some other therapeutic products comprise a sodium perborate gel, which can be placed in the pocket adjuvantly after SRP for oxygen release. However, sodium perborates are known to be corrosive and irritating substances, which, despite their antibacterial effects, are cytotoxic and thus impair tissue regeneration. Another approach relates to compositions comprising hyaluronic acid, which have been described in the context with the treatment of gum diseases.

DE20201602375U1 discloses hyaluronic acid in combination with the antiseptic octenidine dihydrochloride, which is suitable for the treatment and prevention of several diseases such as stomatological complications, gum injuries after calculus removal, oral mucosal wounds or tongue wounds, or of other types of wounds and injuries in the oral cavity.

EP1638582B1 discloses the use of hyaluronic acid for the preparation of compositions for the treatment of recurrent oral aphthae.

EP3056195A1 discloses a composition of hyaluronic acid and beta-glucan and its use for the treatment of, among others, periodontitis, and gingivitis.

WO2018/158764A1 discloses a certain composition of a polyalkylene oxide block copolymer and hyaluronic acid and its use for the treatment of, among others, periodontitis, gingivitis, and peri-implantitis.

Furthermore, root fillings are known in which CaO₂ is used as an antimicrobial agent (Malyk Yuriy (2005), "In vitro studies on the use of calcium peroxide (CaO2)-containing sealer materials in endodontic therapy", doctoral thesis, Poliklinik für Zahnerhaltung und Parodontologie der Ludwig-Maximilians-Universitat München).

WO2018/026204A1 discloses an in-situ crosslinked polymer hydrogel comprising calcium peroxide that releases a high concentration of oxygen in a sustained release form. Also described is an implant material for tissue regeneration and filling, comprising such an in-situ crosslinked sustained release oxygen-release hydrogel.

CN112826761A and CN112870109A disclose a preparation method of a dentifrice with antibacterial effect comprising calcium peroxide.

Although various compositions for the treatment of the various gum diseases in humans or animals are known, they do not combine a long-lasting antibacterial effect, while at the same time gum tissue regeneration is supported, making the treatment of gingivitis, periodontitis, and peri-implantitis with the known compositions less efficient.

### Summary of the Invention

Against this background, it is the object of the present invention to provide a composition, which unites both an anti-bacterial effect and at the same time mediates tissue regeneration in order to improve the therapeutic outcome of gum diseases, such as gingivitis, periodontitis, and peri-implantitis.

This object is solved by the subject-matter of claim 1, in particular by a composition comprising hyaluronic acid or a pharmaceutically acceptable salt thereof, and calcium peroxide (CaO₂).

The present invention is based on the surprising discovery that oxygen is released from CaO₂ in a delayed manner when the latter is combined with hyaluronic acid in a composition. Mixing both components together results in a continuous and steady release of anti-bacterial oxygen for a prolonged period during treatment of a gum disease, thereby making the treatment more efficient. The inventive combination is in particular suitable for the prevention or treatment of gingivitis, periodontitis, and peri-implantitis, since calcium peroxide exhibits unexpected properties when combined with hyaluronic acid or a pharmaceutically acceptable salt thereof. That said, the delayed release of oxygen from calcium peroxide is most probably attributable to the matrix formed by the hyaluronic acid said composition.

It was further found by the inventors that due to the delayed release of oxygen from hyaluronic acid-bound calcium peroxide, otherwise harmful concentrations of oxygen for the surrounding gum tissue are avoided. Periodontal pockets provide an ideal habitat for anaerobic pathogenic germs due to their anaerobic conditions. Furthermore, inflamed tissue increases gingival crevicular fluid, the major nutritional source for subgingival microorganisms.

As a further advantage of the composition of the present invention, the effective period of oxygen to destroy biofilms and bacteria in the periodontal pockets is significantly increased, so that the treatment of the gum disease becomes more effective. As a consequence, tissue breakdown can be avoided or, at least, retarded or stopped. At the same time, the tissue regeneration is stimulated by hyaluronic acid and not adversely affected by the action of calcium peroxide.

The beneficial effect of the release of oxygen in a delayed manner when calcium peroxide is combined with hyaluronic acid in a composition for use in the treatment of a gum disease could not be expected. In fact, it would have been more likely that oxygen is released in the same manner as in water since hyaluronic acid only forms a rather coarse meshed matrix. It was hence surprising that a combination of hyaluronic acid and calcium peroxide would result in a delayed release of oxygen without adversely affecting the surrounding tissue, while at the same time the antimicrobial properties of calcium peroxide are maintained. As a result, the growth of periodontal pathogenic bacteria or biofilm formation and immune inflammatory responses can be effectively inhibited. Most importantly, no toxic effects could be observed on fibroblasts or osteoblasts.

As shown in the present invention, the basic causes of periodontitis or peri-implantitis can be solved by (a) increasing the antimicrobial efficiency against obligate anaerobes, thus restoring a symbiotic bacterial flora, and (b) by acceleration of tissue regeneration to prevent recolonization by bacteria in periodontal pockets.

The compositions of the present invention are preferably formulated as a pharmaceutical composition. Preferably, the pharmaceutical composition comprises one or more pharmaceutically acceptable carriers, solvents, diluents, or expedients, and is preferably prepared as a formulation for topical administration in the oral cavity.

Preferably, the compositions of the present invention are formulated in form of a solution, a suspension, an emulsion, a cream, or a gel. Such formulations contain a therapeutically effective concentration of hyaluronic acid or a pharmaceutically acceptable salt thereof, and a therapeutically effective concentration of calcium peroxide (CaO₂). In a preferred embodiment, the composition of the present invention is provided as a gel, which is ready to be injected into the space between the teeth and gums with a delivery means, e.g. a syringe. Due to the inventive combination, the gel has a bactericidal effect against pathogenic periodontal germs, prevents new colonization after mechanical cleaning and accelerates tissue regeneration (e.g. bone, gums). In addition, the gel temporarily closes the gap between the teeth and the gums, in which pathogenic bacteria otherwise would multiply.

The composition of the present invention preferably includes sterile aqueous solutions, or dispersions and sterile powders for the preparation of sterile injectable solutions or dispersions. The injectable solutions must be sterile and fluid to the extent that they can be injected by means of any suitable delivery means for the oral cavity, such as a syringe. The injectable solution preferably comprises a carrier, which can be any suitable solvent or dispersion medium containing, for example, water, ethanol, polyol (e.g. glycerol, propylene glycol and liquid polyethylene glycol), suitable mixtures thereof, or vegetable oils. The effective dosage of active ingredient employed may vary depending on the particular compound employed, the mode of administration, and the severity of the condition being treated. However, in general, satisfactory results are obtained when the compounds of the invention are administered at a single dosage of up to 3mL per application.

Preferred dosage forms suitable for administration to the oral cavity comprise from about 15 mg/mL to 35 mg/mL of hyaluronic acid and from about 100_{[AB1]} mg/L to 500 mg/L calcium peroxide in admixture with a pharmaceutically acceptable carrier. This dosage regimen may be adjusted to provide the optimal therapeutic response. The inventive combination can be administered to an oral cavity of a human or an animal suffering from a gum disease. Typically, the veterinary dosages correspond to human dosages with the amounts administered being in proportion to the weight of the animal as compared to adult humans.

For example, for administration to the oral cavity, a pharmaceutically acceptable carrier which does not adversely affect the activity of hyaluronic acid and calcium peroxide, such as a solvent or a diluent, may be contained in the pharmaceutical composition of the present invention. Such carriers include, but are not limited to agents, stabilizers, absorption delaying agents, disintegrating agents, emulsifiers, antioxidants, binders, lubricants, moisture absorbents, and the like. For example, the solvent may be selected from the group consisting of water and sucrose solutions, the diluent may be selected from the group consisting of lactose, starch, and microcrystalline cellulose, the absorption delaying agent may be selected from the group consisting of chitosan and aglycosyl glycans, and the lubricant may be selected from the group consisting of magnesium.

In preferred embodiments of the invention, the composition of the present invention preferably further includes a carrier, a thickener, and one or more additional antimicrobial agents. In accordance with various aspects of these embodiments, the composition may include a humectant, migration enhancing agents, oils, and the like.

Preferably, the pharmaceutically acceptable salts of hyaluronic acid are hyaluronan salts and include, but are not limited to alkaline metal or alkaline earth metal salts of hyaluronan, e.g. sodium hyaluronate, potassium hyaluronate and calcium hyaluronate. In one embodiment, the pharmaceutically acceptable salt of hyaluronic acid is sodium hyaluronate. In an alternative embodiment, the pharmaceutically acceptable salt of hyaluronic acid is potassium hyaluronate or calcium hyaluronate. The composition of the invention may also comprise a combination of sodium hyaluronate, potassium hyaluronate and/or calcium hyaluronate.

Preferably, in the compositions of the present invention, the hyaluronic acid or pharmaceutically active salt thereof is provided in form of a matrix to allow binding of calcium peroxide. The hyaluronic acid matrix according to the present invention promotes tissue regeneration and viability of fibroblasts. In addition, the hyaluronic acid matrix prevents calcium peroxide going into solution, and reduces the risk of further depositions of new, potentially pathogenic, germs. When calcium peroxide is bound to the hyaluronic acid matrix, oxygen is released evenly from the calcium peroxide component in the composition, and thereby inhibits the growth of periodontal pathogenic organisms over a long period of time. Example of such periodontal pathogenic organisms include obligate anaerobic germs, in particular periopathogens, which include, but are not limited to *Porphyromonas gingivalis, Aggregatibacter actinomycetemcomitans,* or gram-positive aerobes composed of *Streptococcus spp.* such as *Streptococcus oralis,* and *Actinomyces spp.*

The hyaluronic acid or salt thereof used in the composition of the present invention can be either a high-molecular weight hyaluronic acid, or a low-molecular weight hyaluronic acid, or salts thereof. The high-molecular weight hyaluronic acid or salt thereof preferably has an average molecular weight of about 300,000 to about 6 million Daltons (Da). Most preferably, it has an average molecular weight of from about 500,000 to about 3 million Daltons. The low-molecular weight hyaluronic acid or salt thereof comprised within the composition of the present invention has a molecular weight of up to about 50,000 Da, and is optionally hydrolysed or partially hydrolysed. In certain embodiments, the low-molecular weight hyaluronic acid has a molecular weight of from about 3,000 to about 50,000 Da, from about 4,000 to about 20,000 Da, or from about 6,000 to about 10,000 Da.

In a preferred embodiment, the hyaluronic acid or the pharmaceutically acceptable salt thereof comprised with the composition of the present invention is present in an amount of from about 1 mg/mL to about 100 mg/mL, preferably from about 5 mg/mL to about 80 mg/mL, from about 5 mg/mL to about 50 mg/mL, or from about 10 mg/mL to about 40 mg/mL, more preferably from about 10 mg/mL to about 30 mg/mL, most preferably 20 mg/mL to about 25 mg/mL ,e.g. in an amount of 21 mg/mL, 22 mg/mL, 23 mg/mL, 24 mg/mL or 25 mg/mL.

The calcium peroxide component of the composition of the present invention is included in an amount sufficient to allow even release of oxygen when the composition is applied to the oral cavity. Typically, the calcium peroxide component can be employed in the composition of the present invention in amounts so that at least about 1 mg/L of the composition comprises calcium peroxide.

In a preferred embodiment, the calcium peroxide is present in the composition in an amount of from about 1 mg/L to 5000 mg/L. In a more preferred embodiment, the calcium peroxide comprised with the composition of the present invention is present in an amount of from about 10 mg/L to 1000 mg/L, preferably from about 10 mg/L to about 800 mg/L, from about 100 mg/L to about 800 mg/L, or from about 100 mg/L to about 500 mg/L, more preferably from about 200 mg/L to about 500 mg/L, e.g. in an amount of 200 mg/L, 250mg/L, 300 mg/L, 400 mg/L or 500 mg/L.

A further aspect of the present invention relates to additional components that are preferably present in the composition to increase its efficiency or therapeutic effectivity. In a first aspect of the present invention, the composition comprises one or more additives, which include, but are not limited to plant extracts, calcium hydroxyapatite, and/or collagen. For example, the composition of the invention preferably additionally comprises a preparation of one or more plant extracts, preferably derived from the plant variety selected from the group consisting of eucalyptus, rhubarb root, clove oil, aloe vera, and/or cumin. The presence of plant extracts in the composition of the present invention further inhibits the growth of the germs and constitutes a feasible alternative to antibiotics. As shown herein, the plant extracts furthermore enhance the antibacterial effect of CaO₂ when present in the composition.

In a further preferred embodiment of the present invention, the additive in the inventive composition is calcium hydroxyapatite, which was found to induce the formation and storage of natural collagen. Furthermore, calcium hydroxyapatite promotes the reconstruction of the teeth and bone material.

In a further preferred embodiment of the present invention, the additive in the inventive composition is collagen. Collagen supports the adhesion of the gums to the teeth when present in the composition of the present invention.

When the composition comprises one or more additives selected from the group consisting of plant extracts, calcium hydroxy apatite, or collagen, the plant extract is present in the composition of the present invention in an amount of from about 0.1% to about 10% by weight, more preferably in an amount of from about 0.3% to about 3% by weight, most preferably in a concentration of 0.5% by weight.

In a further preferred embodiment, the calcium hydroxyapatite is present in the composition of the present invention in a ratio of 0.8 to 2.0 for calcium/phosphate and an amount from about 1% to about 30% by weight, more preferably in an amount of from about 5% to about 20% by weight, most preferably in a concentration of 10% by weight.

In a further preferred embodiment of the present invention, the collagen is present in the composition of the present invention in an amount of from about 0.9% to about 25% by weight, more preferably in an amount of from about 5% to about 20% by weight, most preferably in a concentration of 15% by weight.

The invention also comprises variants or modified derivatives of the compounds described herein, in particular chemically or biologically modified forms of hyaluronic acid, or its salts, calcium peroxide, plant extracts, calcium hydroxy apatite or collagen.

A pH value of the composition may vary in accordance with a particular application. In accordance with various embodiments of the invention, the pH value is between about 4 and about 9, preferably the pH is between about 6 and 8.5, most preferably between 6.5 and 8.

The compositions of the present invention do not only exhibit a bactericidal effect on the periodontal pathogenic germs and temporarily prevent their resettlement, but they also actively support tissue regeneration. In particular, the matrix of hyaluronic acid enables a promoted release of oxygen over a prolonged period of time with a simultaneous positive effect on tissue regeneration. Thereby, the compositions of the present invention lead to a higher success rate for the treatment of a gum disease, and reduce the likelihood of a re-treatment. It is apparent for a person skilled in the art that due to the general properties, the compositions of the present invention may be suitable for the treatment of any gum disease but is in particular useful for the treatment or prevention of gingivitis, periodontitis or peri-implantitis.

The invention furthermore comprises a kit for delivering a composition into a gingival pocket, a periodontal pocket or a pocket resulting from peri-implantitis, or for applying said composition on or into the oral mucosa. The kit of the present invention comprises a composition according to the present invention, and a delivery means for topically applying the composition into the gingival pocket, periodontal pocket or pocket resulting from peri-implantitis, or for applying the composition on or into the oral mucosa. In a preferred embodiment, in the kit of the present invention, the composition is provided in form of a gel which is preferably applied to the oral cavity with a syringe. Preferably, the gel is provided in a gingival pocket, a periodontal pocket, or a pocket that has been formed as a result of peri-implantitis. In a preferred embodiment, the delivery means for the composition in the kit is a syringe. In an alternative embodiment, the composition of the invention is applied on or into the oral mucosa. The composition in the kit of the invention allows a sustained release dosage form that can be administered to an oral cavity of an animal or human.

Taken together, the compositions and kits of the present invention allow a delayed release of oxygen from the antimicrobial-acting calcium peroxide, whereby gum diseases such as gingivitis, periodontitis and peri-implantitis, or other gum disease-related inflammatory diseases can be successfully treated without adversely affecting the surrounding tooth or implant tissue.

The invention is explained in more detail in the following examples, which, however, are intended to be of illustrative nature only. By no means shall the herein described examples and embodiments limit the scope of the invention. The invention also comprises any variation, modification, and/or combination of components described herein.

### Brief description of the Figures

**Figure 1** shows the cytotoxicity of hyaluronic acid, calcium peroxide, and a combination thereof in human primary fibroblasts *in vitro.*
**Figure 2** shows the cytotoxicity of hyaluronic acid, calcium peroxide, and a combination thereof in human primary osteoblasts *in vitro.*
**Figure 3** shows the cytotoxicity of hyaluronic acid, calcium peroxide, and a combination thereof in human primary umbilical vein endothelial cells (HUVEC) *in vitro.*
**Figure 4** shows the biocompatibility of hyaluronic acid, calcium peroxide, and a combination thereof in a chorioallantoic membrane assay (Figure 4A: Vascularized Surface of the Sponge; Figure 4B: Degradation of the Sponge).
**Figure 5** shows the effect of calcium peroxide on planktonic bacteria colonies of *Streptococcus oralis (S. oralis)* and *Porphyromonas gingivalis (P. gingivalis).*
**Figure 6** shows the effect of hyaluronic acid and calcium peroxide on mature P. *gingivalis* biofilms.
**Figure 7** shows the effect of hyaluronic acid and calcium peroxide on mature S. *oralis* biofilms.
**Figure 8** shows the slow oxygen release from a combination of hyaluronic acid and calcium peroxide compared to calcium peroxide alone and hyaluronic acid alone.

### Modes for carrying out the invention:

### Suitable formulations for application into the oral cavity:

The following non-limiting examples describe formulations that are suitable for the treatment of a number of gum diseases, including but not limited to gingivitis, periodontitis and peri-implantitis, or other gum disease-related inflammatory diseases. The formulations can further comprise any suitable carrier or excipient known to the person skilled in the art. The dosage regimens may be adjusted to provide the optimal therapeutic response. All formulations exhibit the desired therapeutic effect.

### Example 1:

The formulation comprises 256 mg/L calcium peroxide suspended in phosphate buffered saline (PBS) to stabilize the pH in the range of 7 and 7.5. Then, 23 mg/mL hyaluronic acid or salt thereof is added under vigorous stirring until a homogenous gel is produced.

### Example 2:

The formulation comprises 256 mg/L calcium peroxide suspended in phosphate buffered saline (PBS) to stabilize the pH in the range of 7 and 7.5. Then 23 mg/mL hyaluronic acid or salt thereof and 4 mg/L *Rheum palmatum* root extract is added under vigorous stirring until a homogenous gel is produced.

### Example 3:

The formulation comprises 256 mg/L calcium peroxide suspended in phosphate buffered saline (PBS) to stabilize the pH in the range of 7 and 7.5. Then 23 mg/mL hyaluronic acid or salt thereof and 100 mg/L calcium hydroxy apatite is added under vigorous stirring until a homogenous gel is produced.

### Example 4:

The formulation comprises 500 mg/L calcium peroxide suspended in phosphate buffered saline (PBS) to stabilize the pH in the range of 7 and 7.5. Then 30 mg/mL hyaluronic acid or salt thereof and 100 mg/L calcium hydroxy apatite is added under vigorous stirring until a homogenous gel is produced.

### Example 5:

The formulation comprises 256 mg/L calcium peroxide suspended in phosphate buffered saline (PBS) to stabilize the pH in the range of 7 and 7.5. Then 23 mg/mL hyaluronic acid or salt thereof and 10 mg/L collagen is added under vigorous stirring until a homogenous gel is produced.

### Examination of the tissue compatibility of the substances used in the invention

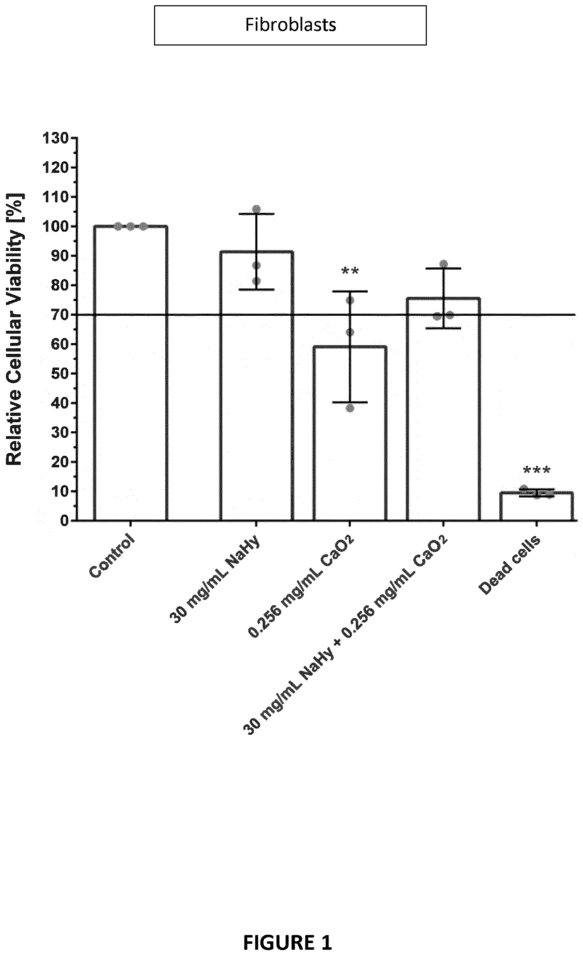

To investigate the cytotoxicity of calcium peroxide, hyaluronic acid, and a combination thereof on cells, the substances were given to human primary fibroblasts, osteoblasts, and human umbilical vein endothelial cells (HUVEC) *in vitro* and compared to untreated cells and a dead control. For this purpose, cytotoxicity tests were performed according to ISO 10993-5 by measuring the cell viability quantitatively and calorimetrically. Additionally, biocompatibility tests were performed in the chorioallantoic membrane assay (CAM assay) in fertilized hen's eggs. All tests were repeated at least three times independently. In all tests 30 mg/mL hyaluronic acid (NaHy) and 0.256 mg/mL CaO₂ were used. According to ISO 10993-5, cytotoxicity is defined by more than 30% reduction of the viable cell number by the substance. The results are shown in Figures 1 to 4.

Figure 1 shows the cellular viability of human primary fibroblasts obtained from human oral mucosa. Fibroblasts were treated with hyaluronic acid, or a combination of calcium peroxide and hyaluronic acid. Both treatments hardly showed a reduction of the cellular viability in comparison to untreated fibroblasts (=100% cell viability). This indicates that neither hyaluronic acid alone nor in combination with calcium peroxide is cytotoxic. By contrast, fibroblasts treated with 0.256 mg/mL calcium peroxide showed a reduction in cell viability by more than 30%, indicating the cytotoxicity of calcium peroxide as single substance.

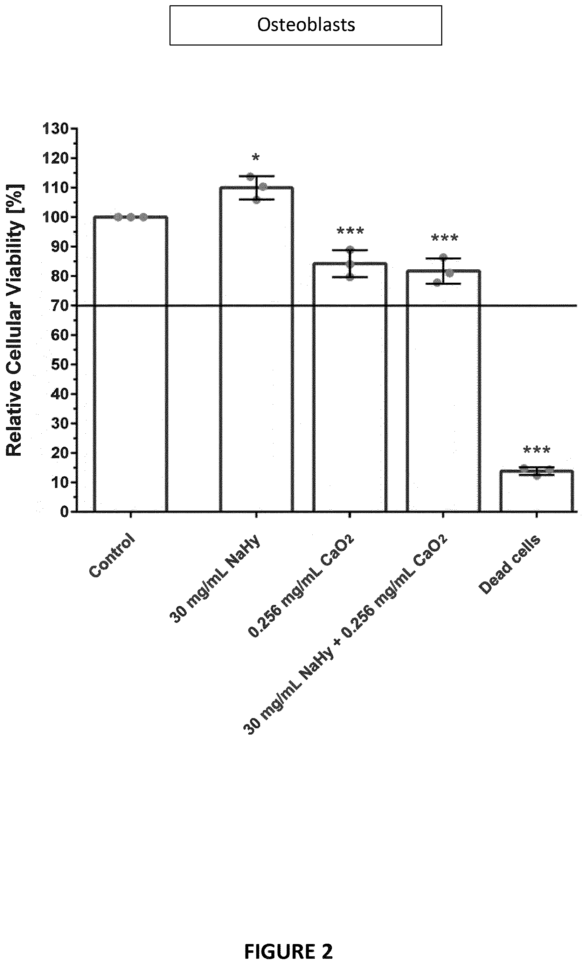

Figure 2 shows the cellular viability of human primary osteoblasts. Osteoblasts were treated with hyaluronic acid, calcium peroxide, or a combination thereof. Osteoblasts hardly showed a reduction of their viability after treatment in comparison to untreated osteoblasts (=100% cell viability). This indicates that both substances, as single substances and in combination, have no cytotoxic effect on osteoblasts.

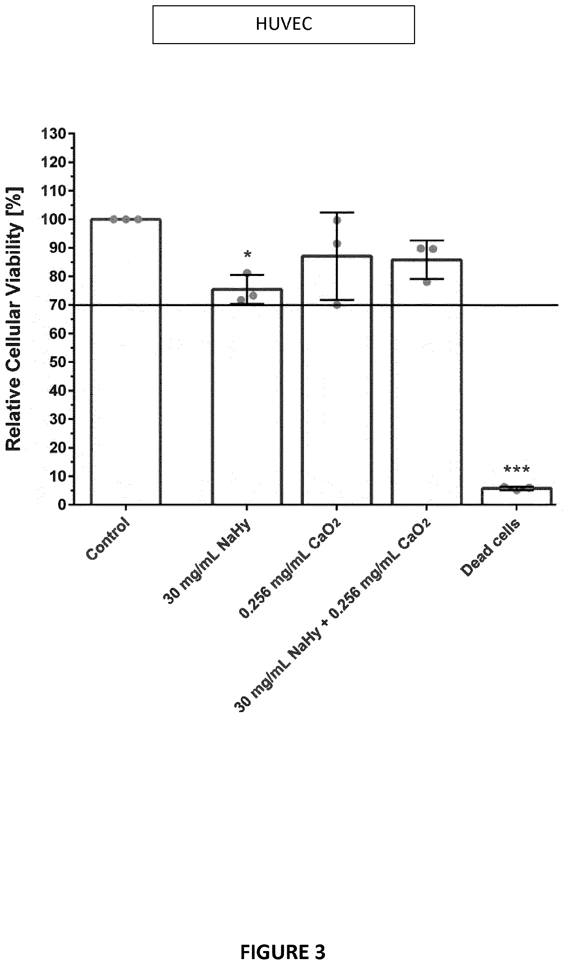

Figure 3 shows the cellular viability of human primary umbilical vein endothelial cells (HUVECs). Both substances, even in combination, had no cytotoxic effects on HUVECs. The viability of HUVECs was slightly increased by the combination of both substances in comparison to treatment with hyaluronic acid as single substance.

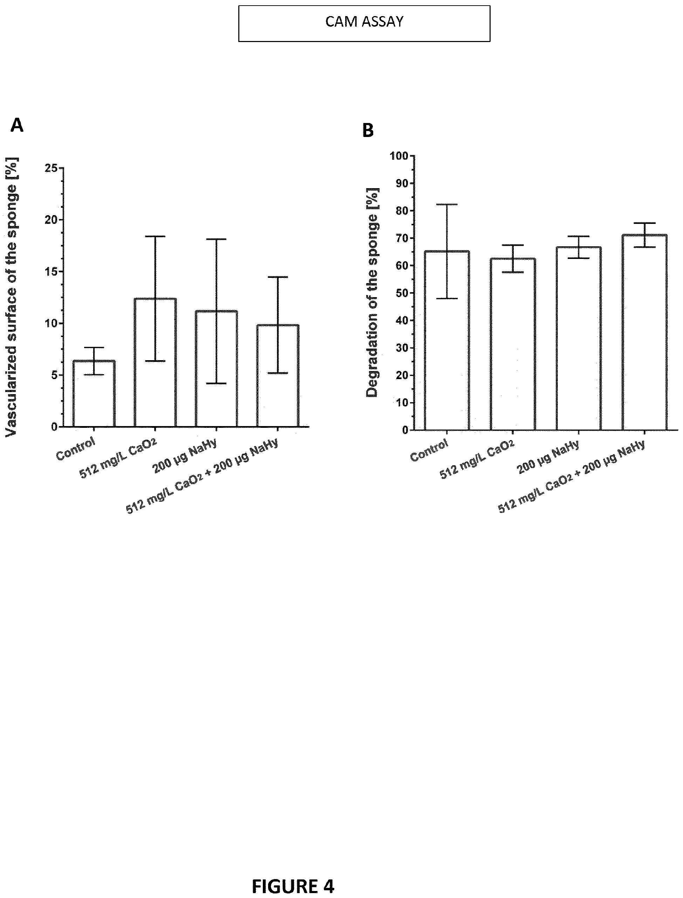

To investigate the biocompatibility of hyaluronic acid, calcium peroxide, and a combination thereof, a CAM assay was performed (Figure 4). The data of Figure 4A and 4B demonstrates that none of the tested substances had a cytotoxic effect on the CAM. The degradation of the sponge on which the different substances were applied was not different from the degradation of an empty sponge and no adverse tissue reactions to the substances were seen on the CAM. Angiogenesis, measurable in the difference of the area of the sponge that is covered by blood vessels was not significantly different in the CAM tissue treated with hyaluronic acid, calcium peroxide, or a combination thereof compared to untreated CAM tissue.

In summary, the combination according to the invention has been shown to be non-cytotoxic in all investigated cell types and in the CAM assay it proved to be biocompatible.

### Inhibitory efficacy of calcium peroxide on planktonic bacteria

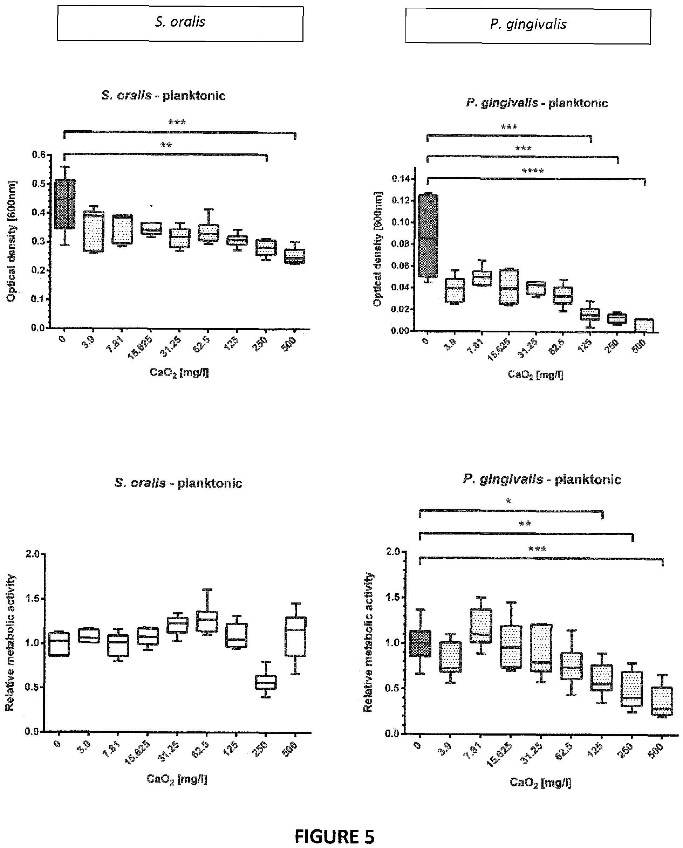

To demonstrate the inhibitory efficacy of calcium peroxide on planktonic bacteria, a selection of calcium peroxide concentrations was utilized. According to Figure 5, different concentrations of calcium peroxide were applied to mature biofilms in combination with hyaluronic acid. Calcium peroxide was added to planktonic bacterial strains S. *oralis* and *P. gingivalis* in concentrations ranging from 0 mg/L (control) to 500 mg/L with hyaluronic acid being present in a concentration of 11.550 mg/L. Shown in each case are the optical density at 600 nm, a parameter for the growth of a bacterial culture, and the relative metabolic activity. At concentrations above 125 mg/L, the optical density decreased, showing that the growth of the periodontitis-causing bacterium P. *gingivalis* was significantly reduced.

In the Figure 5, upper left part, there is shown that the optical density of a planktonic bacterium of the physiological oral flora, S. *oralis,* decreased significantly at calcium peroxide concentrations of 250 mg/L and 500 mg/L compared to the control. In the lower left part, it is shown that the relative metabolic activity of S. *oralis* was unchanged compared to the control. In the Figure 5, top right part, it is shown that the optical density of the periodontitis-causing bacterium P. *gingivalis* significantly decreased at calcium peroxide concentrations of 125 mg/L, 250 mg/L, and 500 mg/L compared to the control. In the lower right part, it is shown that the relative metabolic activity of P. *gingivalis* significantly decreased at calcium peroxide concentrations of 125 mg/L, 250 mg/L, and 500 mg/L compared to the control.

These results show that a calcium peroxide concentration of 125 mg/L has a statistically significant inhibitory effect on the periodontitis-causing bacterium P. *gingivalis,* but not on S. *oralis,* a gram-positive, facultatively anaerobic bacterium of the streptococcus family which is found in the physiological oral flora. At concentrations of around 500 mg/L a stress response is induced in S. *oralis* by increasing concentrations of calcium peroxide, whereas a stress response in periodontal pathogenic bacterium P. *gingivalis* is already induced at significantly lower concentrations. However, at concentrations of greater than 125 mg/L, the oxygen showed a toxic effect regarding P. *gingivalis.* On the other hand, the growth of bacteria of the commensal flora, as presented by S. oralis, is only very slightly inhibited by concentrations of 125 mg/L. The data indicate a selective inhibition of the harmful bacteria, while the physiological flora remains largely unaffected.

### Inhibitory efficacy of calcium peroxide on biofilms

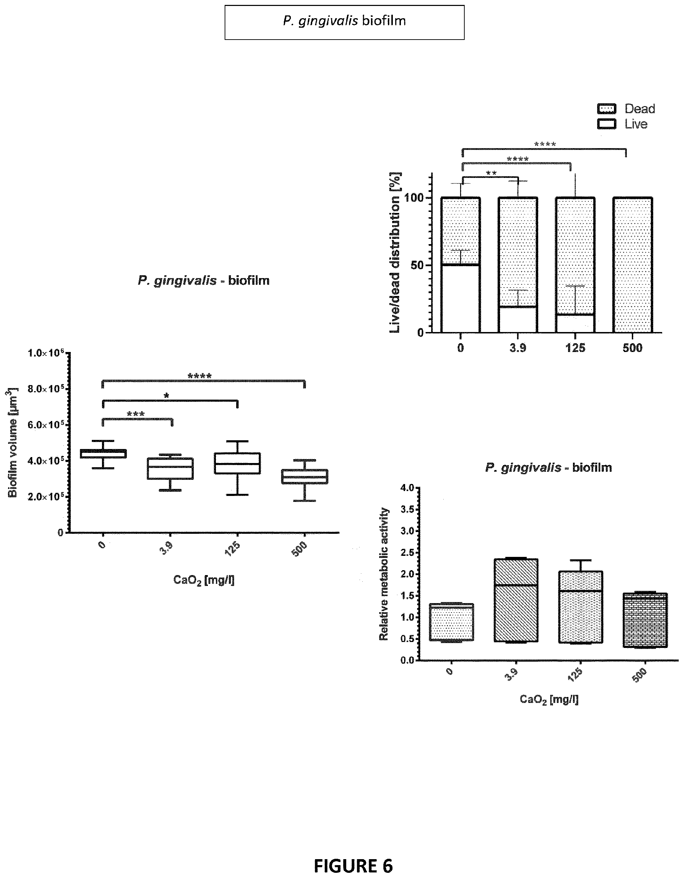

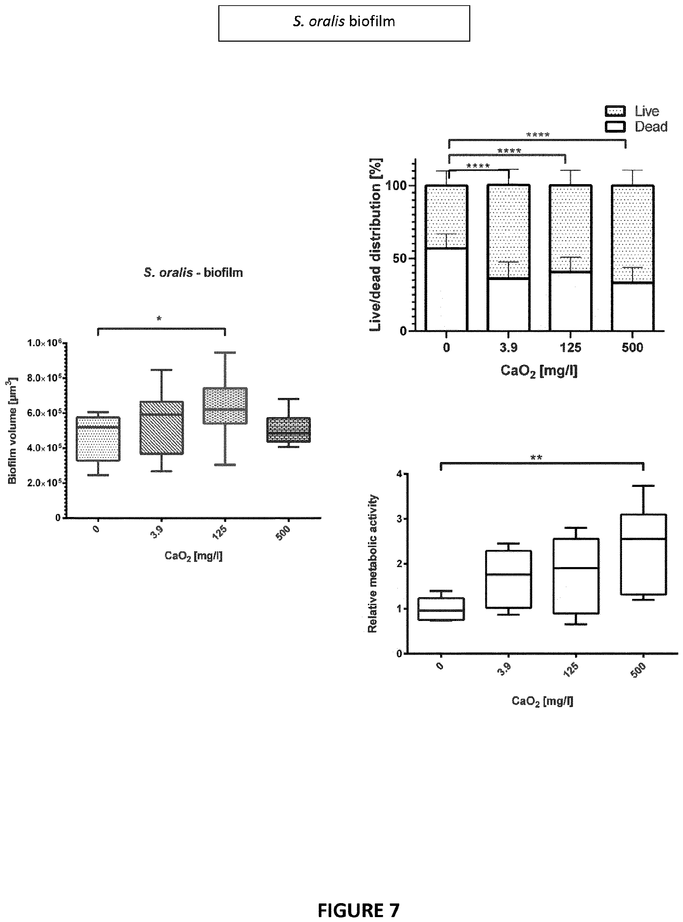

To show the effect of a combination of calcium peroxide and hyaluronic acid on biofilms, different concentrations were utilized and the biofilm volume, percentage of alive and dead cells as well as the relative metabolic activity of the periodontitis-causing bacterium *P. gingivalis* and the physiological bacterium of the oral flora S. *oralis* were measured. The results are shown in Figure 6 and 7.

Figure 6 shows the effect of hyaluronic acid and calcium peroxide on mature P. *gingivalis* biofilms. In the Figure 6, left part, it is shown that the biofilm volume [µm³] decreased significantly at calcium peroxide concentrations of 3.9 mg/L, 125 mg/L, and 500 mg/L compared to the control. In Figure 6, top right part, it is shown that the percentage of dead bacteria increased significantly at calcium peroxide concentrations of 3.9 mg/L, 125 mg/L, and 500 mg/L compared to the control. In addition, the percentage of dead bacteria increased significantly at calcium peroxide concentration of 500 mg/L compared with 3.9 mg/L and 125 mg/L. In the figure below right, it is shown that metabolic activity shows no significant difference.

Figure 7 shows the effect of hyaluronic acid and calcium peroxide on mature S. *oralis* biofilms. In the Figure 7, left part, it is shown that the biofilm volume [µm³] increased significantly at a calcium peroxide concentration of 125 mg/L compared to the control.

In the Figure 7, top right part, it is shown that the percentage of dead bacteria increased significantly at calcium peroxide concentrations of 3.9 mg/L, 125 mg/L, and 500 mg/L compared to the control. In the Figure 7, lower right part, it is shown that the metabolic activity increased significantly at a calcium peroxide concentration of 500 mg/L compared to the control.

The results indicate that application to biofilms according to the invention leads to a significant inhibition of the growth of the anaerobic periodontitis-causing bacterium P. *gingivalis* by calcium peroxide concentrations of 3.9, 125 and 500 mg/L in hyaluronic acid. In contrast, aerobic bacteria of the physiological oral flora, such as S. *oralis,* are merely affected by the oxygen released from the matrix.

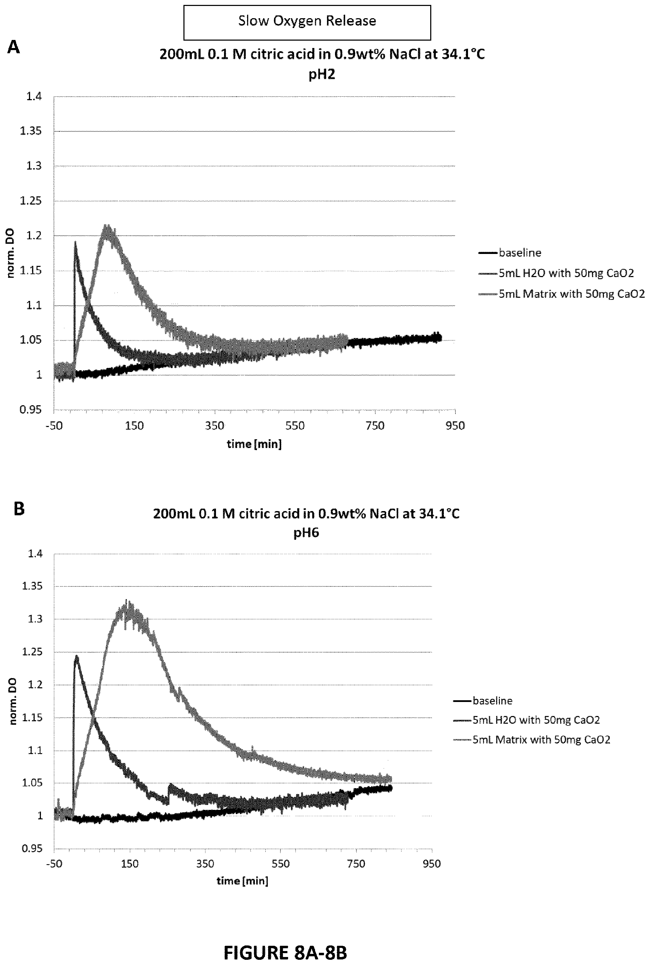

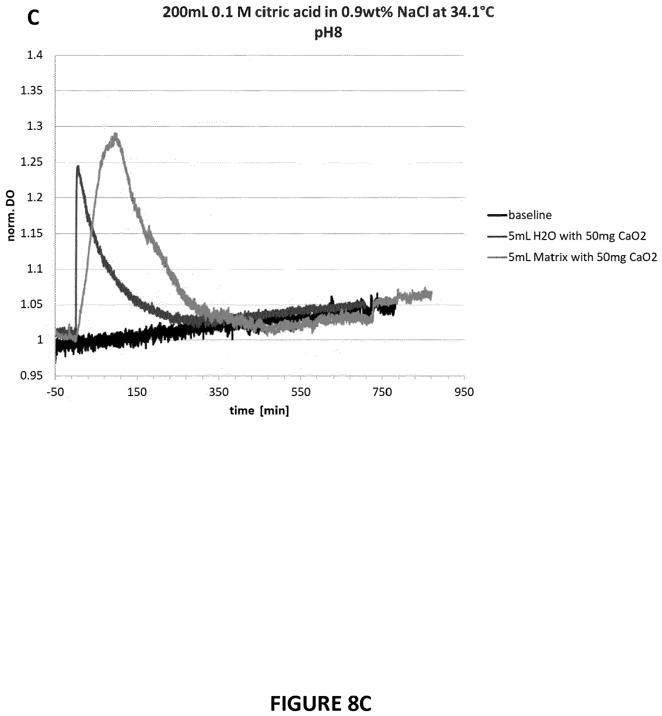

Figure 8 shows the slow oxygen release effect of the combination of hyaluronic acid and calcium peroxide compared to calcium peroxide or hyaluronic acid only. It is shown that the release of oxygen increased significantly if calcium peroxide is enclosed in a hyaluronic acid matrix. Furthermore, the combination of hyaluronic acid and calcium peroxide increased the amount of available oxygen as indicated by a larger area under the curve (AUC). The experiments were conducted at different pH levels (Fig. 8A at pH 2, Fig. 8B at pH 6, and Fig. 8C at pH 8) with either 50 mg calcium peroxide solved in 5 mL aqua (H₂O) or 5 mL hyaluronic acid matrix compared to baseline over a time period of 850 min.

In summary, the results indicate that the combination of calcium peroxide and hyaluronic acid effectively minimizes the growth of periodontic anaerobes without affecting the physiological flora. Because calcium peroxide is trapped in the hyaluronic acid matrix, oxygen is released in a delayed manner, such that only non-harmful concentration oxygen reaches the cells through the hyaluronic acid matrix. At the same time, tissue regeneration is promoted and not adversely affected.

### Material and Methods

### Preparations of calcium peroxide and hyaluronic acid

Calcium peroxide (Sigma Aldrich, St. Louis, MO, US) was suspended in water or buffered aqueous solution. The desired amount of hyaluronic acid sodium salt (HERRLAN-PSM e.K.) was added during vigorous stirring.

### Preparations of calcium peroxide and hyaluronic acid for bacterial assays

The autoclaved calcium peroxide (CaO₂) (Sigma Aldrich, St. Louis, MO, US) was dissolved in sterile filtered, deionized, and autoclaved water. By serial dilutions with water (for planktonic experiments) or BHI/VitK (for biofilm experiments) the solutions were adjusted to concentration of 3.9 - 500 mg/l CaO₂ for bacterial experiments. All solutions were freshly prepared for each experiment.

### Cell Isolation

Primary human cells were obtained from patients who underwent surgery at the University Medical Center Mainz, Germany. The use of residual materials was approved by the ethics committee of the Landesarztekammer Rheinland-Pfalz in accordance with the principles expressed in the Declaration of Helsinki and the International Harmonized Guidelines for good clinical practice (ICH-GCP). All patients provided written consent.

Fibroblasts were obtained from human oral mucosa. Tissue samples were cut into small pieces of approximately 2x2 mm with a sterile disposable scalpel. Prior to cell isolation, the tissue pieces were stepwise sterilized in 70% ethanol, in sterillium^{®} classic pure (Bode Chemie GmbH, Hamburg, Germany), and again in 70% ethanol. Then they were transferred to 5-10 mL (depending on the amount of tissue) 0.5% protease solution (P6141, Sigma-Aldrich, St. Louis, MO, US) in phosphate buffered saline (PBS; Sigma-Aldrich, St. Louis, MO, US) and incubated overnight at 4°C. The next day, the protease solution was incubated while shaking for further 15 min at 37°C. The sample was then passed through a cell sieve (EASYstrainerTM 70 µm sterile, Greiner bio-one, Kremsmunster, Austria) with the help of a cell scraper (Falcon^{®}, Corning, NY, US). Cells were pelleted by centrifugation (1,500 rpm, 5 min), were transferred to cell culture medium, and seeded into small cell culture flasks with a grow area of 25 cm². Cells were characterized morphologically and were used at most until passage 10 to ensure primary identity. Cells were maintained in DMEM/Ham's F12 (Gibco, ThermoFisher Scientific, Waltham, MA, USA) supplemented with 10% fetal calf serum and antibiotics (10,000 U/mL penicillin and 10 mg/mL streptomycin; Sigma-Aldrich, St. Louis, MO, USA) at 37°C in 5% CO₂.

Primary human osteoblasts were isolated according to the following protocol. Human bone specimens were obtained during hip or knee joint replacement surgeries. The use of residual materials was approved by the ethics committee of the Landesärztekammer Rheinland-Pfalz in accordance with the principles expressed in the Declaration of Helsinki and the International Harmonized Guidelines for good clinical practice (ICH-GCP). All patients provided written consent.

Cancellous bone fragments were removed with bone rongeurs from bone specimens. The isolated fragments were washed several times with PBS (Sigma-Aldrich, St. Louis, MO, USA) until a clear supernatant was achieved. The supernatant was discarded and 15 mL collagenase type I solution (1 mg/mL in medium 199) was added. Collagenase digestion was carried out under mechanical stirring in a water bath at 37 °C. After 45 min, the fragments were washed again several times with PBS (Sigma-Aldrich). The washed bone pieces were transferred into 6-well tissue culture plates with sterile forceps, followed by addition of DMEM/F-12 medium supplemented with 20% fetal calf serum (FCS) and 1% penicillin/streptomycin (PS).

After the first passage, human osteoblasts were cultured in DMEM/Ham's F12 (Gibco, ThermoFisher Scientific, Waltham, MA, USA) supplemented with 10% fetal calf serum and antibiotics (10,000 U/mL penicillin and 10 mg/mL streptomycin; Sigma-Aldrich, St. Louis, MO, USA). The medium was changed twice a week. For osteoblast differentiation, the medium was supplemented with 10 nM dexamethasone, 3.5 mM b-glycerophosphate, and 50 µg/mL ascorbic acid.

Primary Human Umbilical Vein Endothelial Cells (HUVEC) were isolated from the vein of the umbilical cord. The use of residual materials was approved by the ethics committee of the Landesärztekammer Rheinland-Pfalz in accordance with the principles expressed in the Declaration of Helsinki and the International Harmonized Guidelines for good clinical practice (ICH-GCP). All patients provided written consent. The umbilical cord was flushed with PBS (Sigma-Aldrich) until the buffer was clear and blood clots in the vein were removed. Then, a collagenase solution was injected into the umbilical cord, and it was placed in warm PBS and incubated for 12 min at 37 °C. After incubation, the collagenase solution containing the endothelial cells was flushed from the cord perfusing the vein with PBS (Sigma-Aldrich). The effluent was collected and centrifuged for 10 min at room temperature with max. 420 g. Cells were kept in culture dishes coated with 3 mL 0.1% gelatin at 37°C for the first cell passages. Cells were incubated overnight at 37°C and washed with PBS (Sigma-Aldrich) the next day. Endopan 3 with 3% fetal blood serum (PAN Biotech, Aidenbach, Germany) was used as culture cell medium.

### Cellular Viability Assays

Cells were seeded into a 24-well-plate and they were given time to adhere overnight. The cell number per well was 50.000 for fibroblasts and HUVECs and 40.000 for osteoblasts each with respectively 1.5 mL medium per well. After 24 h, cell culture medium was replaced by 400 µl fresh medium. Cells were treated with 0.256 mg/mL CaO₂ (Sigma Aldrich, St. Louis, MO, USA), or 30 mg/mL hyaluronic acid (Herrlan PSM e.K.) or a combination thereof. Untreated cells served as control. The substances were applied into inserts with a 10 µm thick translucent polycarbonate membrane (Corning Inc., New York, USA) with 0.4 µm pores. Those inserts then were inserted in the 24-well-plate and were thus in direct contact with the cell culture medium of the cells and incubated overnight. The inserts were removed after 24 h, the cell culture medium was exchanged for 1 mL of medium with 10% AlamarBlue^{™} Cell Viability Reagent (ThermoFisher Scientific, Waltham, MA, USA) and the cells were incubated for 4 h at 37°C. After 4 h, the liquid (200 µl per well) was transferred from the 24-well-plate to a black 96-well-plate (Greiner bio-one GmbH, Frickenhausen) for measurements. The AlamarBlue assay is based on the change of the blue color of the non-fluorescent indicator dye (resazurin) after acceptance of electrons, which, passing from the oxidized state to the reduced state, becomes a fluorescent pink compound. Fluorescence was measured on a Fluorescence Microplate Reader (Fluoroskan Ascent Microplate reader, ThermoFisher Scientific, Waltham, MA, USA). Results were given as relative fluorescence using a 538 nm excitation filter and a 600 nm emission filter, normalized to untreated control.

### Chorio-allantoic membrane assay (CAM Assay)

Fertilized white Leghorn chicken eggs (LSL Rhein-Main GmbH, Dieburg, Germany) were incubated at 38°C with constant humidity of 55 rH in an incubator (Type 3000 digital and fully automatic, Siepmann GmbH, Herdecke, Germany). For the first three days, eggs were placed horizontally on one side to ensure that the CAM would detach from the upwards pointing eggshell. On the embryonic development day (EDD) 3, eggs were prepared by removing 5-6 mL of the albumen in order to enlarge the space between eggshell and CAM. A small window of 3x2 cm was cut into the upwards pointing part of the eggshell. The window was covered with Parafilm^{®} (Sigma-Aldrich, St. Louis, MO, USA) to prevent evaporation. On EDD-8, Gelaspon Strips (Bausch & Lomb Inc.; New York, USA) were cut in slices of 1x0.5x2 mm. 20 µl of each substance (512 mg/L CaO₂, 10 mg/mL NaHY, combination thereof) were added to the sponge. 20 µl of water was used as control. The further assessment of potential tissue adverse events was performed blinded. After 3 h, on EDD-10, and EDD-12, pictures were taken with a digital intravital fluorescence microscope (Olympus BXFM, Olympus Deutschland GmbH, Hamburg, Germany) at a 100-fold magnification using the cellSens Dimension software package.

### Microorganisms and culture conditions

*Streptococcus oralis* ATCC 9811 was obtained from the American Type Culture Collection (ATCC) and *Porphyromonas gingivalis* DSM 20709 derived from the German Collection of Microorganisms and Cell Cultures (DSMZ). The strains were grown under anaerobic conditions (80% N₂, 10% H₂, 10% CO₂) at 37°C for 24 h in 50 mL screw cap tube (Sarstedt, Nümbrecht, Germany) with Brain Heart Infusion Medium (BHI; Oxoid, Wesel, Germany) containing 10 µg/mL vitamin K (Roth, Karlsruhe, Germany; BHI/VitK) to obtain 24-hour-old precultures for further processing.

### Investigation of the planktonic bacterial growth and metabolic activity of S. oralis and P. gingivalis under the influence of CaO₂

In order to determine the influence of CaO₂ on planktonic S. *oralis* and *P. gingivalis,* the 24-hour-old precultures were pelleted by centrifugation at 4,000 g for 15 min at 4°C, the supernatant was discarded and the bacterial pellets were resuspended in fresh double concentrated BHI/VitK medium (2x BH/VitK). The optical density was measured at 600 nm (OD₆₀₀; BioPhotometer, Eppendorf, Hamburg, Germany), adjusted to 0.2 and dilute 1:10 with 2x BHI/VitK to 0.02. The freshly prepared CaO₂ solution was mixed equally with the bacterial cultures (OD₆₀₀ = 0.02) to a final OD₆₀₀ = 0.01 and following final concentrations for CaO₂:

| | | | | | | | | | | |
|---|---|---|---|---|---|---|---|---|---|---|
| CaO₂ [mg/l] | - | - | 3.90 | 7,8125 | 15.625 | 31.25 | 62.5 | 125 | 250 | 500 |

As positive controls, bacterial cultures (OD₆₀₀ = 0.02) were mixed 1:2 with sterile filtered, deionized and autoclaved water (growth controls). As negative controls, 2x BHI/VitK was mixed equally with sterile filtered, deionized and autoclaved water (medium control) and the CaO₂ solution was mixed 1:2 with 2x BHI/VitK (CaO₂ solution control). Finally, 150 µL of each suspension was transferred into each well of a 96-well plate and cultivated for 24 h at 37°C rotating (180 rpm) under anaerobic conditions (Anaerobic Jar and AnaeroGen; Oxoid, Wesel, Germany). All experiments were carried out in three biological and two technical replicates.

To evaluate the effect of CaO₂ on the growth of planktonic S. *oralis* and P. *gingivalis* cultures, the 24-h-old bacterial cultures were mixed and the optical density (OD₆₀₀) was measured with a plate reader (Tecan, Mennedorf, Switzerland). Metabolic activity was measured using the BacTiter-Glo^{™} Microbial Viability Assay (Promega, Mannheim, Germany). 50 µL BacTiter-Glo^{™} reagent and 50 µL of the well-mixed bacterial cultures were added to opaque 96-well plates. After 5 min of incubation at room temperature under light protection, the samples were mixed again and the amount of adenosine triphosphate (ATP) was determined by measuring the luminescence using the plate reader. All results were normalized to the medium or CaO₂ solution controls.

### Investigation of CaO₂ effect on S. oralis and P. gingivalis biofilms

To evaluate the effects of CaO₂ on biofilms of S. *oralis* and P. *gingivalis,* 24-h-old pre-cultures were processed as described for the planktonic experiments, except that 1x BHI/VitK medium was used as well as OD₆₀₀ was adjusted to 0.1 and diluted to 0.01. A volume of 2 mL of each bacterial culture (OD₆₀₀ = 0.01) was added to each well of a 6-well plate and cultivated for 24 h at 37°C under anaerobic conditions (Anaerobic Jar and AnaeroGen; Oxoid, Wesel, Germany). All experiments were carried out in three biological and two technical replicates.

After 24 h of growth, pH value was measured of all samples and controls with indicator sticks (PH-FIX indicator sticks pH 0-14; Macherey-Nagel, Duren, Germany). Supernatants were removed, 2 mL fresh BHI/VitK was added to the controls, and 2 mL of CaO₂ solution was added to the biofilms that should be treated with following concentrations:

| | | | |
|---|---|---|---|
| CaO₂ [mg/l] | 3.90 | 125 | 500 |

After two hours of incubation under anaerobic conditions (Anaerobic Jar and AnaeroGen; Oxoid, Wesel, Germany) at 37°C, the pH values of all samples and controls were measured again. The metabolic activity was determined using the BacTiter-Glo Microbial Viability Assay (Promega, Mannheim, Germany). Biofilms were rinsed twice with Phosphate Buffered Saline (PBS; Biochrom GmbH, Berlin, Germany) and 1 mL BacTiter-Glo reagent was added to each biofilm. The biofilms rinsed off with the reagent by pipetting up and down several times. After 5 min of incubation under rotation (180 rpm) and light protection, 100 µL of the solutions were added to opaque 96-well plates. Samples were mixed again and the luminescence was measured with the plate reader (Tecan, Mennedorf, Switzerland). In order to analyze the effect of CaO₂ on biofilm volume and live/death distribution, the LIVE/DEAD BacLight Bacterial Viability Kit (Life Technologies, Carlsbad, California, USA) was used to stain the biofilms with SYTO 9 and propidium iodide according to the manufacturer's recommendations. Subsequently, the biofilms were rinsed twice with PBS and fixed with 2.5% glutardialdehyde (Roth, Karlsruhe, Germany) in PBS for 30 min. After fixation, the biofilms were washed twice and covered with 3 mL PBS. They were microscopically analyzed using a confocal laser scanning microscope (CLSM; Leica TCS SP8, Leica Microsystems, Mannheim, Germany) with excitation and emission wavelengths for SYTO 9 of 488 nm and 500-550 nm and for propidium iodide of 552 nm and 675-750 nm. Three images were taken from each biofilm using a z-step of 2 µm. 3D image processing and analysis of the biofilm volumes and proportions of dead and viable bacteria was performed using Imaris x64 software (version 8.4.1, Bitplane AG, Zurich, Switzerland).

### Oxygen Release Measurements

50 mg CaO₂ (75% 200 mesh, Sigma Aldrich) was suspended in 5 mL deionized water (millipore grade). 115 mg of hyaluronic acid sodium salt (1,5 - 2,5MDa: Herrlan-PSM, Alpen, Germany) were added under vigorous stirring. The mixture was stirred for at least 4 h and filled without bubbles into a syringe.

The concentration profile of dissolved oxygen was determined by means of a HI 5421 (Hanna Instruments, Vöhringen, Germany). The sensor was placed in a thermostated beaker right above a magnetic stirring bar. The beaker was filled with 200 mL of 0.1M citric acid (≥99,5%: Carl Roth, Karlsruhe, Germany) in 0.9wt% NaCl aqueous solution. The pH was adjusted by means of 1M NaOH (Carl Roth). A cellulose filter bag was placed into the solution to enable free migration of dissolved substance but avoiding the free migration of the hyaluronic acid matrix or undissolved CaO2 particles.

The detector started to detect the concentration of dissolved oxygen (DO mode) and the baseline was detected for ca. 50 min. Then 5 mL of the test solution were added into the cellulose filter bag within 30 s. The concentration profile of the dissolved oxygen was detected for several hours at base line and with 50 mg CaO₂ in 5 mL water either with or without hyaluronic acid sodium salt. The concentration of the dissolved oxygen was normalized to the concentration of the dissolved oxygen at the time when samples was added.

### Statistical analysis

Graphic processing and statistical analysis was performed using the GraphPad Prism software 8.4 (GraphPad Software Inc., La Jolla, USA). To determine whether the data are normally distributed, the Kolmogorov-Smirnov normality test was applied. If data were normally distributed, Ordinary One-Way ANOVA with Dunnett's correction for multiple comparisons was used for to determine statistically significant differences of treated samples compared to the controls. If data failed normal distribution assumption, Kruskal-Wallis test with Dunn's correction for multiple comparisons was used. The significance level was set to p ≤ 0.05 for all comparisons.

## Claims

1. A composition comprising hyaluronic acid or a pharmaceutically acceptable salt thereof, and calcium peroxide (CaO₂) for use in the prevention or treatment of a gum disease.

2. The composition according to claim 1, comprising one or more additives selected from the group consisting of
a) plant extracts,
b) calcium hydroxy apatite,
c) collagen.

3. The composition according to claim 2, wherein said plant extracts are obtained from eucalyptus, rhubarb root, clove oil, aloe vera and/or cumin.

4. The composition according to any one of claims 1 to 3, wherein the composition is composed of a gel, or liquid.

5. The composition according to any one of claims 1 to 4, wherein said gum disease is selected from gingivitis, periodontitis or peri-implantitis.

6. The composition according to any one of claims 1 to 5, wherein said calcium peroxide is present in said composition in an amount of from about 1 mg/L to 5000 mg/L.

7. The composition according to any one of claims 1 to 6, wherein said calcium peroxide is present in the composition in an amount of from about 20 mg/L to 1000 mg/L.

8. The composition according to any one of claims 1 to 7, wherein said hyaluronic acid or said pharmaceutically acceptable salt thereof is present in said composition in an amount of from about 1 mg/mL to about 100 mg/mL.

9. The composition according to any one of claims 2 to 8, wherein said plant extract is present in said composition in an amount of from about 0.1% to about 10% by weight.

10. The composition according to any one of claims 2 to 9, wherein said calcium hydroxyapatite is present in said composition in an amount of from about 1% to about 30% by weight.

11. The composition according to any one of claims 2 to 9, wherein said calcium hydroxy apatite is present in said composition in a ratio of 0.8 to 2.0 for calcium/phosphate.

12. The composition according to any one of claims 2 to 11, wherein said collagen is present in said composition in an amount of from about 0.9% to about 25% by weight.

13. The composition according to any one of claims 1 to 12, wherein the pH of the composition is in a range of from 6 to 8.5.

14. The composition according to any one of claims 1 to 13, wherein said composition is formulated as a solution, a suspension, an emulsion, a cream, or a gel.

15. A kit for delivering a composition into a gingival pocket, a periodontal pocket or a pocket resulting from peri-implantitis, or for applying said composition on or into the oral mucosa, said kit comprising a composition according to any one of claims 1 to 14, and a delivery means for topically applying said composition into said gingival pocket, periodontal pocket or pocket resulting from peri-implantitis, or for applying said composition on or into said oral mucosa.
